(19) Europäisches Patentamt
European Patent Office
Office européen des brevets

(11) Veröffentlichungsnummer: **0 116 842**
**A2**

(12)

# EUROPÄISCHE PATENTANMELDUNG

(21) Anmeldenummer: 84100314.8

(22) Anmeldetag: 13.01.84

(51) Int. Cl.[3]: C 07 C 103/52
A 61 K 37/02

(30) Priorität: 22.01.83 DE 3302125

(43) Veröffentlichungstag der Anmeldung:
29.08.84 Patentblatt 84/35

(84) Benannte Vertragsstaaten:
AT BE CH DE FR GB IT LI LU NL SE

(71) Anmelder: BOEHRINGER INGELHEIM KG
D-6507 Ingelheim am Rhein(DE)

(84) Benannte Vertragsstaaten:
AT BE CH DE FR GB IT LI LU NL SE

(71) Anmelder: Boehringer Ingelheim International G.m.b.H
D-6507 Ingelheim am Rhein(DE)

(84) Benannte Vertragsstaaten:
GB

(72) Erfinder: Schnorrenberg, Gerd, Dr.
Fichtenweg 13
D-6507 Ingelheim/Rhein(DE)

(72) Erfinder: Roos, Otto, Dr.
Elsheimer Strasse 36
D-6501 Schwabenheim(DE)

(72) Erfinder: Lösel, Walter, Dr.
Im Herzenacker 26
D-6535 Gau-Algesheim(DE)

(72) Erfinder: Wiedemann, Ingrid, Dr.
Ernst-Ludwig-Strasse 61
D-6535 Gau-Algesheim(DE)

(72) Erfinder: Gaida, Wolfram, Dr.
Paul-Clemen-Strasse 14
D-6507 Ingelheim/Rhein(DE)

(72) Erfinder: Hoefke, Wolfgang, Dr.
Rosselstrasse 21
D-6200 Wiesbaden(DE)

(54) **Aminosäure-Derivate, Verfahren zu ihrer Herstellung und Verwendung.**

(57) Die Erfindung betrifft neue Aminosäure-Derivate der allgemeinen Formel

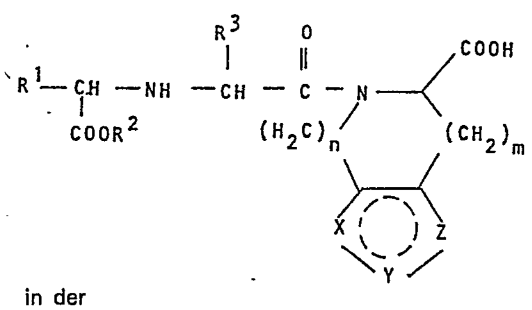

(I)

in der

$R^1$ Wasserstoff, eine Alkylgruppe mit 1 bis 6 Kohlenstoffatomen oder eine Phenylalkylgruppe mit 7 bis 12 Kohlenstoffatomen,

$R^2$ Wasserstoff, eine Alkylgruppe mit 1 bis 4 Kohlenstoffatomen oder eine Phenylalkylgruppe mit 7 bis 12 Kohlenstoffatomen,

$R^3$ Wasserstoff oder eine Alkylgruppe mit 1 bis 6 Kohlenstoffatomen,

n und m jeweils 0, 1 oder 2, wobei die Summe aus n und m 1 oder 2 ist,

X, Y und Z Sauerstoff, Schwefel, $NR^4$, $CR^5$, $CHR^5$,

$$-\overset{R^5}{\overset{|}{CH}}-\overset{R^5}{\overset{|}{CH}}- \text{ oder } -\overset{R^5}{\overset{|}{C}}=\overset{R^5}{\overset{|}{C}}-$$

bedeutet, mit der Maßgabe, daß nur einer der Reste X, Y und Z Sauerstoff, Schwefel,

$$-\overset{R^5}{\overset{|}{CH}}-\overset{R^5}{\overset{|}{CH}}- \text{ oder } -\overset{R^5}{\overset{|}{C}}=\overset{R^5}{\overset{|}{C}}-$$

und ein oder zwei der Reste X, Y und Z $NR^4$ bedeuten können,

$R^4$ Wasserstoff oder eine Alkylgruppe mit 1 - 4 Kohlenstoffatomen und

./...

EP 0 116 842 A2

Croydon Printing Company Ltd.

$R^5$ Wasserstoff oder zusammen mit einem vicinal stehenden Rest $R^5$ einen Phenylring bedeutet oder für m und n = 1, deren Dihydroform mit der Doppelbindung in Konjugation zur C-terminalen Carboxylgruppe und deren Salze, Verfahren zu ihrer Herstellung, pharmazeutische Zusammensetzungen, die eine oder mehrere Verbindungen der allgemeinen Formel I als Wirkstoff enthalten und ihre Verwendung in Arzneimitteln.

Die neuen Aminosäure-Derivate besitzen eine langanhaltende blutdrucksenkende Wirkung, die auf einer Hemmung des Angiotension I Converting Enzyms beruht.

Gegenstand der Erfindung sind Aminosäure-Derivate der allgemeinen Formel I und deren Salze sowie Verfahren zu ihrer Herstellung, ihre pharmazeutischen Zusammensetzungen und ihre Anwendung als Arzneimittel.

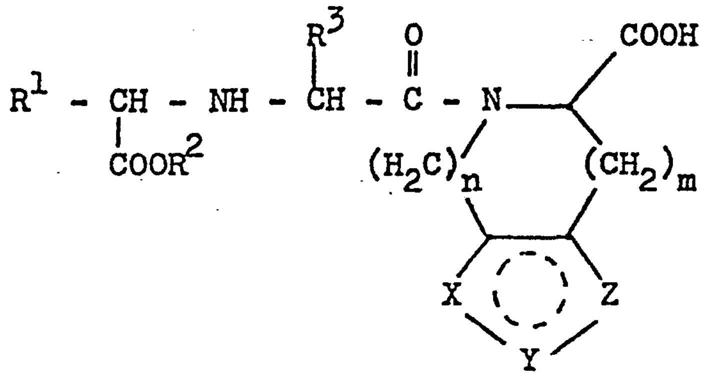

(I)

In dieser Formel bedeuten:

$R^1$ Wasserstoff, eine Alkylgruppe mit 1 bis 6 Kohlenstoffatomen oder eine Phenylalkylgruppe mit 7 bis 12 Kohlenstoffatomen,

$R^2$ Wasserstoff, eine Alkylgruppe mit 1 bis 4 Kohlenstoffatomen oder eine Phenylalkylgruppe mit 7 bis 12 Kohlenstoffatomen,

$R^3$ Wasserstoff oder eine Alkylgruppe mit 1 bis 6 Kohlenstoffatomen,

n und m jeweils 0,1 oder 2, wobei die Summe aus n und m 1 oder 2 ist,

X, Y und Z Sauerstoff, Schwefel, $NR^4$, $CR^5$, $CHR^5$,

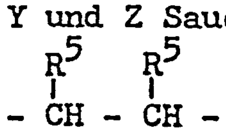 oder 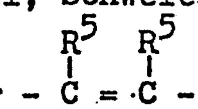, mit der Maßgabe, daß nur einer der Reste X, Y und Z O, S,

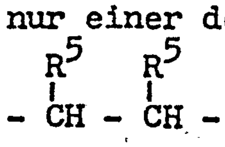 oder 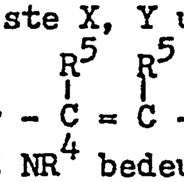 und ein oder zwei der Reste X, Y und Z $NR^4$ bedeuten können,

$R^4$ Wasserstoff oder eine Alkylgruppe mit 1 bis 4 Kohlenstoffatomen und

$R^5$ Wasserstoff oder zusammen mit einem vicinal stehenden Rest $R^5$ einen Phenylring, oder für m und n = 1, deren Dihydroform mit der Doppelbindung in Konjugation zur C-terminalen Carboxylgruppe.

Die an die Pyrrolidin- beziehungsweise Piperidincarbonsäure ankondensierten Fünf- oder Sechsringheterocyclen können gesättigt oder ungesättigt sein. Bevorzugte Heterocyclen sind: Furan, Pyrrol, Thiophen, Benzofuran, Indol, Benzothiophen, Oxazol, Imidazol, Thiazol, Isoxazol, Pyrazol, Pyrrolidin, Tetrahydrofuran, Tetrahydrothiophen, Pyridin, Pyridazin, Chinolin, Isochinolin oder Piperidin.

Die neuen Verbindungen weisen im allgemeinen mehrere Asymmetriezentren auf und liegen daher als Diastereomere oder in Form ihrer Racemate beziehungsweise ihrer racemischen Gemische vor. Die Erfindung umfaßt sowohl die racemischen Gemische als auch die einzelnen Diastereomeren. Bevorzugt sind diejenigen Enantiomeren, bei denen die asymmetrischen C-Atome in der L-Konfiguration vorliegen.

Die Verbindungen der Formel I können als innere Salze oder, falls freie Carboxylgruppen vorhanden sind, als Alkali- oder Erdalkalisalze z.B. als Natrium-, Kalium-, Magnesium- oder Calciumsalz und als physiologisch unbedenkliche Salze mit Aminen wie Trimethylamin oder Dicyclohexylamin vorliegen. Ferner kann eine vorhandene freie Aminogruppe mit einer Mineralsäure, wie Salzsäure oder Bromwasserstoffsäure, oder einer organischen Säure, beispielsweise Essigsäure, zu einem Salz umgesetzt werden.

Die neuen Stoffe der allgemeinen Formel I können nach verschiedenen Verfahren erhalten werden:

a) Durch Umsetzung einer Verbindung der allgemeinen Formel II

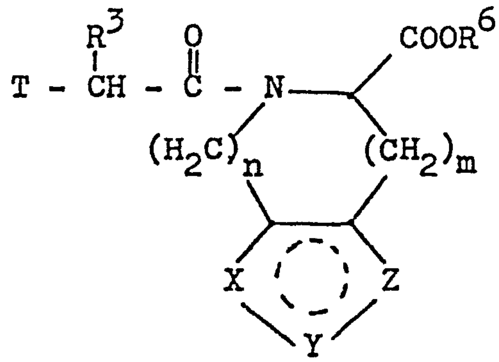

(II)

mit einer Verbindung der allgemeinen Formel III

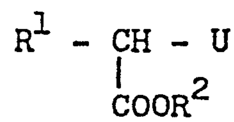

(III)

in denen

T eine nucleofuge Gruppe und
U eine Aminogruppe oder umgekehrt T eine Aminogruppe und U eine nucleofuge Gruppe,
$R^6$ Wasserstoff, eine Alkylgruppe mit 1 bis 4 Kohlenstoffatomen, eine Benzylgruppe oder eine Trimethylsilylgruppe bedeuten und die Reste
$R^1$, $R^2$, $R^3$, n, m, X, Y und Z die oben angeführte Bedeutung besitzen.

Als Nucleofuge können Halogenide dienen, so daß man beispielsweise jeweils von 2-Halogencarbonsäurederivaten und 2-Aminosäurederivaten ausgehen kann. Die Reaktion wird bevorzugt in polaren Lösungsmitteln wie Wasser, Alkoholen, Dimethylformamid, Acetonitril, Dimethylsulfoxid oder deren Gemischen, gegebenenfalls in Anwesenheit von Alkali- oder Erdalkalicarbonaten, tertiären Aminen, quartären Ammoniumhydroxiden oder Tetraalkylguanidinen durchgeführt. Nach Abspaltung von $R^6$ nach den üblichen Verfahren, beispielsweise durch saure oder alkalische Verseifung des Esters oder katalytische Hydrogenolyse, werden die Endprodukte der allgemeinen Formel I erhalten.

b) Durch Umsetzung eines α-Oxocarbonsäurederivats der allgemeinen Formel IV,

$$R^1 - \underset{\displaystyle COOR^2}{\underset{|}{C}} = O \qquad (IV)$$

worin $R^1$ und $R^2$ die oben erwähnte Bedeutung haben, mit einem unter a) beschriebenen Aminosäureamid der allgemeinen Formel III ($U = NH_2$) zum entsprechenden Imin, das reduziert wird. Geeignete Lösungsmittel für diese Reaktion sind Wasser oder Alkohole, aber auch unpolare Lösungsmittel wie Benzol oder Toluol. Bei Verwendung von wasserfreien Lösungsmitteln kann das Reaktionswasser durch Zugabe von Molekularsieb gebunden werden. Die Reduktion kann durch Natriumborhydrid, Natriumcyanborhydrid oder katalytische Hydrierung mit Palladium auf Kohle oder Raney-Nickel als Katalysatoren erfolgen.

Durch Abspaltung von $R^6$ nach den üblichen Verfahren, wie beispielsweise geschildert, werden die Endprodukte der allgemeinen Formel I erhalten.

c) Ausgehend von einer Verbindung der allgemeinen Formel V

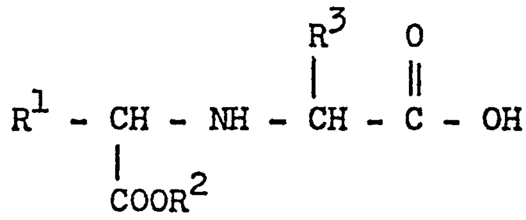

(V)

worin
$R^1$, $R^2$ und $R^3$ die oben angegebene Bedeutung haben, durch Umsetzung mit einer Aminosäure der Formel VI

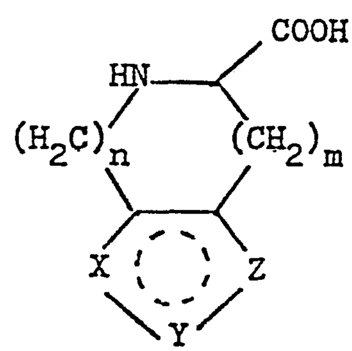

(VI)

worin
die Reste X, Y und Z sowie n und m die oben angegebene Bedeutung haben, beziehungsweise durch Umsetzung mit einem Ester einer Verbindung der allgemeinen Formel VI ($R^6$ anstelle von Wasserstoff).

Die Kondensation kann nach in Houben-Weyl, Methoden der Organischen Chemie, Bd. 15, beschriebenen Methoden erfolgen. Bevorzugtes Kondensationsmittel für die Reaktion ist N,N'-Dicyclohexylcarbodiimid, bevorzugte Carboxylschutzgruppe für die Aminosäure VI ist die tert.-Butyl-, Benzyl- oder Trimethylsilylgruppe. Nach erfolgter Kondensation können diese nach den üblichen Verfahren, wie beispielsweise geschildert, abgespalten werden.

Die Ausgangsverbindungen II (T = Halogen) sind durch Kondensation von Estern von Aminosäuren der allgemeinen Formel VI mit 2-Halogencarbonsäuren über deren entsprechende Säurechloride, gemischten Anhydride, Aktivester oder über andere in Houben-Weyl, Methoden der Organischen Chemie, Bd. 15, beschriebene Methoden zugänglich.

Die Verbindungen II (T = $NH_2$) werden durch Reaktion von Estern der Aminosäuren mit N-geschützten Aminocarbonsäuren erhalten. Als Aminoschutzgruppen und Kondensationsmittel werden in Houben-Weyl, Methoden der Organischen Chemie, Bd. 15, beschriebene verwendet. Bevorzugt als Aminoschutzgruppen werden die Benzyloxycarbonyl- oder die Fluorenylmethoxycarbonylgruppe und als Kondensationsmittel N,N'-Dicyclohexylcarbodiimid verwendet.

Ausgangsverbindungen der allgemeinen Formel VI können, je nach Bedeutung der Reste X, Y und Z, erhalten werden beispielsweise durch Umsetzung von

a) Tryptamin und Glyoxylsäure
(B. T. Ho et al, J. Pharm. Sci. 57, 269-274 (1968))

b) Thiophen-2-ethylamin und Glyoxylsäure
(J. P. Moffrand, Heterocycles 16, 35-37 (1981))

c) Tryptophan und Formaldehyd
(D. G. Harvey et al, J. Chem. Soc. 1941, 153-159)

d) Histidin und Formaldehyd
(M. Cain et al, Heterocycles 19, 1003-1007 (1982))

Bei den vorstehend beschriebenen Verfahren können die Ausgangsverbindungen in Form ihrer racemischen Gemische, ihrer Diastereomeren oder Enantiomeren vorliegen. Liegen die racemischen Gemische vor, können aus den Reaktionsprodukten nach den üblichen Verfahren wie fraktionierte Kristallisation oder chromatographische Verfahren die sterisch einheitlichen Formen angereichert oder rein erhalten werden.

Nach den vorstehend beschriebenen Verfahren können beispielsweise erhalten werden:

N-[N-(1-Methoxycarbonyl-3-phenylpropyl)-L-alanyl]-4,5,6,7-tetrahydro-thieno[3,2-c]pyridin-4-carbonsäure

N-[N-(1-Carboxy-2-phenylethyl)-alanyl]-4,5,6,7-tetrahydro-thieno[3,2-c]pyridin-4-carbonsäure

N-[N-(1-Methoxycarbonyl-3-phenylpropyl)-alanyl]-4,5,6,7-tetrahydro-thieno[2,3-c]pyridin-7-carbonsäure

N-[N-(1-Ethoxycarbonyl-3-phenylpropyl)-L-alanyl]-L-2,3,-4,9-tetrahydro-1H-pyrido[3,4-b]indol-3-carbonsäure

N-[N-(1-Methoxycarbonyl-3-phenylpropyl)-alanyl]-L-2,3,4,9-tetrahydro-1H-pyrido[3,4-b]indol-3-carbonsäure

N-[N-(L-1-Ethoxycarbonyl-3-phenylpropyl)-L-alanyl]-L-2,3,4,9-tetrahydro-1H-pyrido[3,4-b]indol-1-carbonsäure

N-[N-(L-1-Carboxy-2-methylpropyl)-alanyl]-L-2,3,4,9-tetrahydro-1H-pyrido[3,4-b]indol-3-carbonsäure

N-[N-(1-Ethoxycarbonylmethyl)-L-alanyl]-2,3,4,9-tetrahydro-1H-pyrido[3,4-b]indol-1-carbonsäure

N-[N-(L-1-Ethoxycarbonyl-3-phenylpropyl)-L-alanyl]-L-4,5,6,7-tetrahydro-thieno[2,3-c]pyridin-7-carbonsäure

N-[N-(1-Carboxy-3-phenylpropyl)-L-alanyl]-4,5,6,7-tetrahydro-pyrrolo[2,3-c]pyridin-7-carbonsäure

N-[N-(1-Ethoxycarbonyl-3-phenylpropyl)-L-alanyl]-5,6,7,8-tetrahydro-pyrido[4,3-b]pyridin-5-carbonsäure

N-[N-(1-Carboxy-3-phenylpropyl)-L-alanyl]-L-4,5,6,7-tetrahydro-1H-imidazo[4,5-c]pyridin-6-carbonsäure

N-[N-(L-1-Äthoxycarbonyl-3-phenylpropyl)-L-alanyl]-4,5,6,7-tetrahydro-thieno[3,2-c]pyridin-L-4-carbonsäure

N-[N-(L-1-Carboxy-3-phenylpropyl)-L-alanyl]-4,5,6,7-tetrahydro-thieno[3,2-c]pyridin-L-4-carbonsäure

N-[N-(L-1-Äthoxycarbonyl-3-phenylpropyl)-L-alanyl]-4,5-dihydro-thieno[3,2-c]pyridin-6-carbonsäure

N-[N-(L-1-Carboxy-3-phenylpropyl)-L-alanyl]-6,7-dihydro-thieno[2,3-c]pyridin-5-carbonsäure

N-[N-(L-1-Carboxy-3-phenylpropyl)-L-alanyl]-4,5-dihydro-thieno[3,2-c]pyridin-6-carbonsäure

Die neuen Endprodukte der allgemeinen Formel I besitzen eine starke, langanhaltende blutdrucksenkende Wirkung. Diese beruht auf einer Hemmung des Angiotensin I Converting Enzyms und damit einer Blockierung der Bildung des Vasokonstriktors Angiotensin II aus Angiotensin I. Darüber hinaus wirken die neuen Verbindungen hemmend auf das für den Bradykininabbau verantwortliche Enzym Kininase II, das als identisch mit dem oben genannten Converting Enzym gilt. Da Bradykinin eine gefäßerweiternde Wirkung besitzt, wird der blutdrucksenkende Effekt durch diese zusätzliche Wirkung verstärkt. Die durch Bradykinin erzeugte Blutdrucksenkung an normalen Ratten wird durch die neuen Verbindungen verstärkt.

Die Anwendung kann intravenös, subkutan oder peroral erfolgen. Die Dosierung bei peroraler Gabe liegt bei 20 - 200 mg je Einzeldosis. Bei intravenöser Gabe oder bei gleichzeitiger Verabreichung mit Diuretika ist eine Herabsetzung der Dosis angebracht.

Für die Anwendung in der Therapie werden die neuen Verbindungen mit üblichen pharmazeutischen Füll- oder Trägerstoffen, Streck-, Spreng-, Binde-, Gleit-, Dickungs- oder Verdünnungsmitteln gemischt.

Als pharmazeutische Zubereitungsformen kommen zum Beispiel Tabletten, Kapseln, Zäpfchen, Lösungen, Säfte, Emulsionen oder dispersible Pulver in Frage, wobei gewünschtenfalls weitere bekannte Wirkstoffe, z.B. Saluretika, Diuretika und/oder Antihypertonika zugefügt werden können.

Entsprechende Tabletten können beispielsweise durch Mischen des oder der Wirkstoffe mit bekannten Hilfsstoffen, beispielsweise inerten Verdünnungsmitteln, wie Calciumcarbonat, Calciumphosphat oder Milchzucker, Sprengmitteln, wie Maisstärke oder Alginsäure, Bindemitteln, wie Stärke oder Gelatine, Schmiermitteln, wie Magnesiumstearat oder Talk, und/oder Mitteln zur Erzielung des Depoteffektes, wie Carboxypolymethylen, Carboxymethylcellulose, Celluloseacetatphtalat, oder Polyvinylacetat erhalten werden. Die Tabletten können auch aus mehreren Schichten bestehen.

Entsprechend können Dragees durch Überziehen von analog den Tabletten hergestellten Kernen mit üblicherweise in Drageeüberzügen verwendeten Mitteln, beispielsweise Kollidon oder Schellack, Gummi arabicum, Talk, Titandioxid oder Zucker, hergestellt werden. Zur Erzielung eines Depoteffektes oder zur Vermeidung von Inkompatibilitäten kann der Kern auch aus mehreren Schichten bestehen. Desgleichen kann auch die Drageehülle zur Erzielung eines Depoteffektes aus mehreren Schichten bestehen, wobei die oben bei den Tabletten erwähnten Hilfsstoffe verwendet werden können.

Säfte der erfindungsgemäßen Wirkstoffe beziehungsweise Wirkstoffkombinationen können zusätzlich noch ein Süßungsmittel, wie Saccharin, Cyclamat, Glycerin oder Zucker sowie ein geschmacksverbesserndes Mittel, z.B. Aromastoffe, wie Vanillin oder Orangenextrakt, enthalten. Sie können außerdem Suspendierhilfsstoffe oder Dickungsmittel, wie Natriumcarboxymethylcellulose, Netzmittel, beispielsweise Kondensationsprodukte von Fettalkoholen mit Ethylenoxid, oder Schutzstoffe, wie p-Hydroxybenzoate, enthalten.

Injektionslösungen werden in üblicher Weise, z.B. unter Zusatz von Konservierungsmitteln, wie p-Hydroxybenzoaten, oder Stabilisatoren, wie Alkalisalzen der Ethylendiamintetraessigsäure und unter Zugabe geeigneter Lösungsvermittler hergestellt und in Injektionsflaschen oder Ampullen abgefüllt.

Die einen oder mehrere Wirkstoffe beziehungsweise Wirkstoffkombinationen enthaltenden Kapseln können beispielsweise hergestellt werden, indem man die Wirkstoffe mit inerten Trägern, wie Milchzucker oder Sorbit, mischt und in Gelatinekapseln einkapselt.

Die folgenden Beispiele dienen zur näheren Erläuterung der Erfindung:

## Beispiel 1

## N-[N-(1-Methoxycarbonyl-3-phenylpropyl)-alanyl]-L-2,3,4,9-tetrahydro-1H-pyrido[3,4-b]indol-3-carbonsäure

15 g L-2,3,4,9-Tetrahydro-1H-pyrido[3,4-b]indol-3-carbonsäure werden mit 7,5 g Chlortrimethylsilan in 200 ml wasserfreiem Tetrahydrofuran 2 Stunden unter Rückfluß gekocht. Es wird auf 0°C gekühlt, 15 g Triethylamin zugegeben und tropfenweise bei 0°C mit einer Lösung aus 17 g 2-Brompropionsäurechlorid und 50 ml wasserfreiem Tetrahydrofuran versetzt, 1 Stunde bei 0°C und über Nacht bei Raumtemperatur gerührt. Ausgefallenes wird abfiltriert, das Filtrat im Vakuum eingeengt, der Rückstand in Essigester gelöst und mit $KHSO_4$- und $NaHCO_3$-Lösung extrahiert. Die vereinigten $NaHCO_3$-Phasen werden mit 2 n HCl angesäuert und mit Dichlormethan extrahiert. Die vereinigten Dichlormethan-Phasen werden mit gesättigter NaCl-Lösung gewaschen, über $MgSO_4$ getrocknet und im Vakuum eingeengt. Das farblose Öl (18 g) wird in 100 ml wasserfreiem Dimethylformamid mit 11,5 g 2-Amino-4-phenylbuttersäuremethylesterhydrochlorid, 1 g Kaliumjodid und 10 g Triethylamin 24 Stunden bei Raumtemperatur gerührt. Dimethylformamid wird im Vakuum abdestilliert, der Rückstand in Essigester gelöst, mit Wasser, $KHSO_4$-Lösung und Wasser gewaschen, über $MgSO_4$ getrocknet und eingedampft. Der Rückstand wird über Kieselgel chromatographiert (Laufmittel: Dichlormethan, Methanol, Essigester (20 : 1 : 2)).
13,9 g (= 60 % d.Th.) der Titelverbindung können als farbloses Öl isoliert werden.
$^1H$-NMR [$CD_3OD$]: $\delta$ = 1,67 ppm, dd [3H], J = 7 Hz; 2,0 m [2H]; 2,7 t [2H], J = 7 Hz; 3,2 - 3,8 m [4H]; 3,7 s [3H]; 4,5 - 5,5 m [3H]; 6,6 - 7,6 m [9H].

Beispiel 2

N-[N-(1-Methoxycarbonyl-3-phenylpropyl)-alanyl]-4,5,6,7-tetrahydro-thieno[2,3-c]pyridin-7-carbonsäure

Zu einer auf 0°C gekühlten Lösung von 10,8 g 4,5,6,7-Tetrahydro-thieno[2,3-c]pyridin-7-carbonsäure in 60 ml 1 n Natronlauge werden gleichzeitig 10,4 g 2-Brompropionylchlorid und 30 ml 2 n Natronlauge getropft und anschließend 2 Stunden bei Raumtemperatur gerührt. Die Lösung wird mit Essigester extrahiert, die wäßrige Phase mit 2 n HCl angesäuert und mit Dichlormethan extrahiert. Die vereinigten organischen Phasen werden mit gesättigter NaCl-Lösung gewaschen, über $MgSO_4$ getrocknet und eingeengt. Der Rückstand wird in wenig Essigester verrieben und abgesaugt. Die farblosen Kristalle (13,3 g) werden in 80 ml wasserfreiem Dimethylformamid mit 9,6 g 2-Amino-4-phenylbuttersäure-methylesterhydrochlorid, 0,8 g Kaliumjodid und 8,4 g Triethylamin 24 Stunden bei Raumtemperatur gerührt. Dimethylformamid wird im Vakuum abdestilliert, der Rückstand in Essigester gelöst, mit Wasser, $KHSO_4$-Lösung und Wasser gewaschen, über $MgSO_4$ getrocknet und eingeengt. Der Rückstand wird über Kieselgel chromatographiert (Laufmittel: Dichlormethan, Methanol, Essigester (100 : 1 : 10)).
Man erhält 11,3 g (= 63 % d.Th.) der Titelverbindung als farbloses Öl.

MS: m/e = 430 ($M^+$), 412, 327

Beispiel 3

N-[N-(L-1-Ethoxycarbonyl-3-phenylpropyl)-L-alanyl]-L-4,5,6,7-tetrahydro-thieno[2,3-c]pyridin-7-carbonsäure

6,2 g N-Fluorenylmethoxycarbonyl-L-alanin werden in 50 ml wasserfreiem Dichlormethan bei 0°C mit 4,5 g Dicyclohexylcarbodiimid versetzt und nach 15 Minuten 4,7 g 4,5,6,7-Tetrahydro-thieno[2,3-c]pyridin-7-carbonsäure-tert.-butylester zugegeben und weitere 30 Minuten bei 0°C und 6 Stunden bei Raumtemperatur gerührt. Es wird abfiltriert und das Filtrat mit gesättigter $KHSO_4$-Lösung, $NaHCO_3$-Lösung und Wasser gewaschen, über $MgSO_4$ getrocknet und im Vakuum eingedampft. Der ölige Rückstand wird über eine Mitteldruckchromatographie an Merck LiChroprep/Si 60 chromatographiert (Laufmittel: Cyclohexan/Essigester (3 : 1)), wobei eine Auftrennung in die Diastereomeren auftritt. Die vierte Fraktion (4,2 g = 42 % d.Th.) wird in 40 ml Dimethylformamid/Piperidin (4 : 1) 2 Stunden bei Raumtemperatur stehengelassen. Dimethylformamid und Piperidin werden abdestilliert und der Rückstand über Kieselgel chromatographiert (Laufmittel: Toluol, Essigester, Ameisensäure (9 : 5 : 1)). 2,3 g werden in 20 ml wasserfreiem Dimethylformamid mit 2 g 2-Brom-4-phenylbuttersäureethylester, 0,2 g Kaliumjodid und 0,75 g Triethylamin 24 Stunden bei Raumtemperatur gerührt. Dimethylformamid wird im Vakuum abdestilliert, der Rückstand in Essigester gelöst, mit Wasser, $KHSO_4$-Lösung und Wasser gewaschen, über $MgSO_4$ getrocknet und eingedampft. Der Rückstand wird über Mitteldruckchromatographie an Merck LiChroprep/Si 60 chromatographiert. (Laufmittel: Dichlormethan, Methanol, Essigester (100:1:1)). Dabei tritt Auftrennung der Diastereomeren ein. Die entsprechende Fraktion wird in 20 ml 1 n HCl in Eisessig 30 Minuten bei Raumtemperatur gerührt. Eisessig wird im Vakuum abdestilliert und der Rückstand mehrfach mit Toluol nachgedampft. Erhalten werden 1,6 g (=49 % d.Th.) des Hydrochlorids der Titelverbindung. MS: m/e = 426 ($M^+$), 412, 327.

Beispiel 4

N-[N-(L-1-Ethoxycarbonyl-3-phenylpropyl)-L-alanyl]-L-2,3,4,9-tetrahydro-1H-pyrido[3,4-b]indol-1-carbonsäure

40,6 g 2-Brom-4-phenylbuttersäureethylester werden mit 14,6 g L-Alanin-tert.-butylester und 20 g Triethylamin in 200 ml wasserfreiem Dimethylformamid bei Raumtemperatur gerührt. Es wird im Vakuum eingedampft, der Rückstand in Essigester gelöst, mit Wasser gewaschen, über $MgSO_4$ getrocknet und im Vakuum eingedampft. Der Rückstand wird über Kieselgel chromatographiert (Laufmittel: Toluol, Essigester, Ameisensäure (9 : 5 : 1)), wobei Trennung der Diastereomeren auftritt. Nach Abdampfen des Lösungsmittels wird die entsprechende Fraktion mit 50 ml 1 n HCl in Eisessig 30 Minuten bei Raumtemperatur gerührt. Es wird im Vakuum eingedampft und mit Ether verrieben, 12,5 g (= 45 % d.Th.) farblose Kristalle. Diese werden mit 12,2 g 2,3,4,9-Tetrahydro-1H-pyrido[3,4-b]indol-1-carbonsäure-tert.-butylester, 6 g 1-Hydroxybenzotriazol und 9,3 g Dicyclohexylcarbodiimid in 100 ml Dimethylformamid 30 Minuten bei 0°C und 2 Stunden bei Raumtemperatur gerührt. Harnstoff wird abfiltriert, Dimethylformamid im Vakuum abdestilliert, der Rückstand in Essigester aufgenommen, mit Wasser gewaschen, über $MgSO_4$ getrocknet und im Vakuum eingedampft. Der Rückstand wird über Kieselgel chromatographiert (Laufmittel: Cyclohexan, Essigester (3 : 1)). Dabei tritt Trennung der Diastereomeren ein. Die entsprechende Fraktion wird mit 50 ml 1 n HCl in Eisessig 30 Minuten bei Raumtemperatur gerührt, Eisessig abgedampft und der Rückstand mit Toluol nachgedampft. 7 g (= 30 % d.Th.) des Hydrochlorids der Titelverbindung werden so erhalten.

Beispiel 5

N-[N-(1-Carboxy-3-phenylpropyl)-L-alanyl]-L-4,5,6,7-tetrahydro-1H-imidazo[4,5-c]pyridin-6-carbonsäure

11,2 g N-Benzyloxycarbonyl-L-alanin, 11,1 g L-4,5,6,7-Tetrahydro-1H-imidazo[4,5-c]pyridin-1-carbonsäure-tert.-butylester und 11,3 g Dicyclohexylcarbodiimid werden in 100 ml wasserfreiem Dichlormethan 30 Minuten bei 0°C und 6 Stunden bei Raumtemperatur gerührt. Harnstoff wird abfiltriert und das Filtrat mit $KHSO_4$, $NaHCO_3$-Lösung und Wasser gewaschen, über $MgSO_4$ getrocknet und eingedampft. Der Rückstand wird in Eisessig gelöst und mit 1 g Palladium auf Kohle und Wasserstoff hydriert. Eisessig wird abdestilliert, der Rückstand mit Diethylether verrieben und abgesaugt. 11,7 g (= 80 % d.Th.) farblose Kristalle werden mit 28,4 g 2-Oxo-4-phenylbuttersäure in 50 ml Ethanol/Wasser (1 : 1) gelöst und bei Raumtemperatur tropfenweise mit einer Lösung von 8 g Natriumcyanborhydrid in 20 ml Ethanol/Wasser (1 : 1) versetzt. Es wird über Nacht gerührt und über Ionenaustauscher Dowex 50 W x 4 (50 - 100 mesh) gegeben. Es wird mit Ethanol/Wasser (1 : 1) gewaschen und mit Ethanol/Wasser (1 : 1) mit 2 % Pyridin eluiert. 13,3 g (= 73 % d.Th.) des tert. Butylester werden mit 50 ml 1 n HCl in Eisessig 30 Minuten bei Raumtemperatur gerührt, der Eisessig abdestilliert und mit Toluol nachgedampft. 10 g (= 58 % d.Th.) des Hydrochlorids der Titelverbindung vom Fp. 163 - 170°C werden erhalten.

Beispiel 6

N-[N-(L-1-Carboxy-3-phenylpropyl)-L-alanyl]-6,7-dihydro-thieno[2,3-c]pyridin-5-carbonsäure

Zu einer auf 0°C gekühlten Lösung aus 716 mg 6,7-Dihydro-thieno[2,3-c]pyridin-carbonsäuremethylesterhydrochlorid, 1 g N-(L-1-Ethoxycarbonyl-3-phenylpropyl)-L-Alanin, 0,5 g 1-Hydroxybenzotriazol und 620 mg Triethylamin in 50 ml Tetrahydrofuran/Dimethylformamid (1 : 1) werden 650 mg Dicyclohexylcarbodiimid gegeben, 1 Stunde bei 0°C und 24 Stunden bei Raumtemperatur gerührt. Das Lösungsmittel wird im Vakuum abdestilliert und der Rückstand in Essigester aufgenommen. Es wird mit gesättigter $NaHCO_3$-Lösung und 0,001 n HCl gewaschen, über $MgSO_4$ getrocknet und eingeengt. Der Rückstand wird in Acetonitril 24 Stunden bei 0°C stehengelassen, ausgefallener Dicyclohexylharnstoff abfiltriert, eingedampft und der Rückstand über Kieselgel chromatographiert (Laufmittel: Essigester/n-Hexan (1 : 1)). Der so erhaltene N-[N-(L-1-Ethoxycarbonyl-3-phenylpropyl)-L-alanyl]-6,7-dihydro-thieno[2,3-c]pyridin-5-carbonsäuremethylester wird mit 1 n Natronlauge/Acetonitril über Nacht gerührt. Acetonitril wird abdestilliert, die wäßrige Lösung mit Essigester extrahiert, mit 1 n HCl auf pH 3 gestellt und filtriert.
Man erhält 620 mg (= 53 % d.Th.) der Titelverbindung als farblose Kristalle.
Fp.: 154°C.

Beispiel 7

N-[N-(L-1-Carboxy-3-phenylpropyl)-L-alanyl]-4,5-dihydro-thieno[3,2-c]pyridin-6-carbonsäure

Aus 924 mg 4,5-Dihydro-thieno[3,2-c]pyridin-6-carbonsäuremethylesterhydrochlorid, 1,3 g N-(L-1-Ethoxycarbonyl-3-phenylpropyl)-L-alanin, 645 mg 1-Hydroxybenzotriazol und 840 mg Dicyclohexylcarbodiimid werden wie in Beispiel 6 beschrieben 1 g (= 60 % d.Th.) der Titelverbindung als farblose Kristalle erhalten.
Fp.: 161°C.

Beispiel 8

N-[N-(L-1-Ethoxycarbonyl-3-phenylpropyl)-L-alanyl]-4,5-dihydro-thieno[3,2-c]pyridin-6-carbonsäure

456 mg N-[N-(L-1-Ethoxycarbonyl-3-phenylpropyl)-L-alanyl]-4,5-dihydro-thieno[3,2-c]pyridin-6-carbonsäuremethylester (erhalten nach Beispiel 7) werden mit 1 ml 1 n NaOH in Acetonitril/Wasser 48 Stunden bei Raumtemperatur gerührt. Es wird eingedampft, der wäßrige Rückstand mit Essigester extrahiert, mit 1 n HCl angesäuert und mit Essigester extrahiert. Die Essigesterphase wurde getrocknet und eingedampft. Es werden 243 mg (= 55 % d.Th.) der Titelverbindung als farbloses Öl erhalten.

Beispiel 9

N-[N-(L-1-Carboxy-3-phenylpropyl)-L-alanyl]-4,5,6,7-tetrahydro-thieno[3,2-c]pyridin-L-4-carbonsäure

Zu 17,3 g DL-4,5,6,7-Tetrahydro-thieno[3,2-c]pyridin-4-carbonsäuremethylesterhydrochlorid, 23,7 g N-(L-1-Ethoxycarbonyl-3-phenylpropyl)-L-alanin, 11,3 g 1-Hydroxybenzotriazol, 15 g Triethylamin in 300 ml Dimethylformamid/Tetrahydrofuran (1 : 2) werden bei 0°C 18,5 g Dicyclohexylharnstoff gegeben, 1 Stunde bei 0°C und 24 Stunden bei Raumtemperatur gerührt. Es wird filtriert und im Vakuum eingedampft. Der Rückstand wird in Essigester aufgenommen, mit gesättigter $NaHCO_3$-Lösung und 0,001 n HCl gewaschen, getrocknet und eingeengt. Der Rückstand wird in Acetonitril 24 Stunden bei 0°C stehengelassen, ausgefallener Dicyclohexylharnstoff abfiltriert, eingeengt und der Rückstand über Kieselgel (Laufmittel: Essigester/n-Hexan (1 : 1)) chromatographiert. Dabei tritt Diastereomerenauftrennung ein. Die N-[N-(L-1-Ethoxycarbonyl-3-phenylpropyl)-L-alanyl]-4,5,6,7-tetrahydro-thieno[3,2-c]pyridin-L-4-carbonsäuremethylester enthaltende Fraktion wird mit 1 n Natronlauge in Acetonitril 24 Stunden bei Raumtemperatur gerührt. Es wird mit Essigester ausgeschüttelt und mit 1 n HCl auf pH 3 gestellt und abfiltriert. Es werden 13,4 g der Titelverbindung als farbloses Pulver erhalten.
Fp. 166 - 167°C.

Analog Beispiel 9 wurden ferner die folgenden Endprodukte erhalten:

N-[N-(L-1-Carboxy-3-phenylpropyl)-L-alanyl]-4,5,6,7-tetrahydro-thieno[2,3-c]pyridin-L-7-carbonsäure, Fp. 169°C;

N-[N-(L-1-Carboxy-3-phenylpropyl)-L-alanyl]-4,5,6,7-tetrahydro-1H-imidazo[4,5-c]pyridin-L-6-carbonsäure, Fp. 180°C (Zers.) - MS: m/e = 382 ($M^+$-$H_2O$);

N-[N-(L-1-Carboxy-3-phenylpropyl)-L-alanyl]-2,3,4,9-tetrahydro-1H-pyrido[3,4-b]indol-L-1-carbonsäure, Fp. 175 - 176°C;

N-[N-(-L-1-Carboxy-3-phenylpropyl)-L-alanyl]-2,3,4,9-tetrahydro-1H-pyrido[3,4-b]indol-L-3-carbonsäure, Fp. 190°C;

N-[N-(L-1-Carboxy-3-phenylpropyl)-L-alanyl]-4,5,6,7-tetrahydro-thieno[3,2-c]pyridin-L-6-carbonsäure, Fp. 157°C;

N-[N-(L-1-Carboxy-3-phenylpropyl)-L-alanyl]-4,5,6,7-tetrahydro-thieno[2,3-c]pyridin-L-5-carbonsäure, Fp. 155 - 157°C.

Beispiel 10

N-[N-(L-1-Ethoxycarbonyl-3-phenylpropyl)-L-alanyl]-4,5,6,7-tetrahydro-thieno[3,2-c]pyridin-L-4-carbonsäure

Aus 4,6 g N-[N-(L-1-Ethoxycarbonyl-3-phenylpropyl)-L-alanyl]-4,5,6,7-tetrahydro-thieno[3,2-c]pyridin-L-4-carbonsäuremethylester (als Zwischenprodukt bei Beispiel 9 beschrieben) werden analog Beispiel 8 mit 10 ml 1 n Natronlauge 3,1 g (=70 % d.Th.) der Titelverbindung als farbloses Pulver erhalten.
Fp. 133 - 135°C.

Beispiel 11

N-[N-(L-1-Ethoxycarbonyl-3-phenylpropyl)-L-alanyl]-4,5,6,7-tetrahydro-thieno[2,3-c]pyridin-L-7-carbonsäure

Aus 4,6 g N-[N-(L-1-Ethoxycarbonyl-3-phenylpropyl)-L-alanyl]-4,5,6,7-tetrahydro-thieno[2,3-c]pyridin-L-7-carbonsäuremethylester werden analog Beispiel 8 mit 10 ml 1 n Natronlauge 2,4 g (= 55 % d.Th.) der Titelverbindung als farbloses Pulver erhalten.
Fp. 75°C (Zers.).

## Pharmazeutische Anwendungsbeispiele

a) Dragees

1 Drageekern enthält:

| | |
|---|---|
| Wirkstoff gemäß Anspruch 1 | 100,0 mg |
| Milchzucker | 60,0 mg |
| Maisstärke | 35,0 mg |
| Gelatine | 3,0 mg |
| Magnesiumstearat | 2,0 mg |
| | 200,0 mg |

Herstellung:

Die Mischung der Wirksubstanz mit Milchzucker und Maisstärke wird mit einer 10%igen wäßrigen Gelatinelösung durch ein Sieb mit 1 mm Maschenweite granuliert, bei 40°C getrocknet und nochmals durch ein Sieb gerieben. Das so erhaltene Granulat wird mit Magnesiumstearat gemischt und verpreßt. Die so erhaltenen Kerne werden in üblicher Weise mit einer Hülle überzogen, die mit Hilfe einer wäßrigen Suspension von Zucker, Titandioxyd, Talkum und Gummi arabicum aufgebracht wird. Die fertigen Dragees werden mit Bienenwachs poliert.

b) Tabletten

| | |
|---|---|
| Wirkstoff gemäß Anspruch 1 | 100,0 mg |
| Milchzucker | 70,0 mg |
| Maisstärke | 50,0 mg |
| lösliche Stärke | 7,0 mg |
| Magnesiumstearat | 3,0 mg |
| | 230,0 mg |

Herstellung:

Wirkstoff und Magnesiumstearat werden mit einer wäßrigen Lösung der löslichen Stärke granuliert, das Granulat getrocknet und innig mit Milchzucker und Maisstärke vermischt. Das Gemisch wird sodann zu Tabletten von 230 mg Gewicht verpreßt, die je 100 mg Wirkstoff enthalten.

c) Injektionslösungen

| | | |
|---|---|---|
| Wirkstoff gemäß Anspruch 1 | | 50,0 mg |
| Ethanolamin | | 60,0 mg |
| Natriumchlorid | | 20,0 mg |
| destilliertes Wasser | ad | 2 ml |

Herstellung:

Der Wirkstoff und die Hilfsstoffe werden in einer ausreichenden Menge destilliertem Wasser gelöst und mit der erforderlichen Menge Wasser auf die gewünschte Konzentration gebracht. Die Lösung wird filtriert und unter aseptischen Bedingungen in 2 ml Ampullen abgefüllt. Die Ampullen werden sterilisiert und verschlossen. Jede Ampulle enthält 50 mg Wirkstoff.

d) Kapseln

| | |
|---|---|
| Wirkstoff gemäß Anspruch 1 | 100,0 mg |
| Milchzucker | 250,0 mg |
| Maisstärke | 40,0 mg |
| Talk | 10,0 mg |
| | 400,0 mg |

Herstellung:

Wirkstoff, Milchzucker und Maisstärke werden zunächst in einem Mischer und dann in einer Zerkleinerungsmaschine vermengt. Das Gemisch wird nochmals in den Mischer gegeben, gründlich mit dem Talk vermengt und maschinell in Hartgelatinekapseln abgefüllt.

e) Suppositorien

| | |
|---|---|
| Wirkstoff gemäß Anspruch 1 | 0,1 g |
| Kakaobutter (Fp. 36-37°C) | 1,6 g |
| Carnaubawachs | 0,1 g |
| | 1,8 g |

Herstellung:

Kakaobutter und Carnaubawachs werden geschmolzen, gründlich vermengt und auf 45°C abgekühlt. In diese Masse wird der feinpulverisierte Wirkstoff eingerührt. Anschließend wird die Mischung in leicht vorgekühlten Suppositorienformen geeigneter Größe gegossen und abkühlen gelassen.

## Patentansprüche

1. Neue Aminosäure-Derivate der allgemeinen Formel

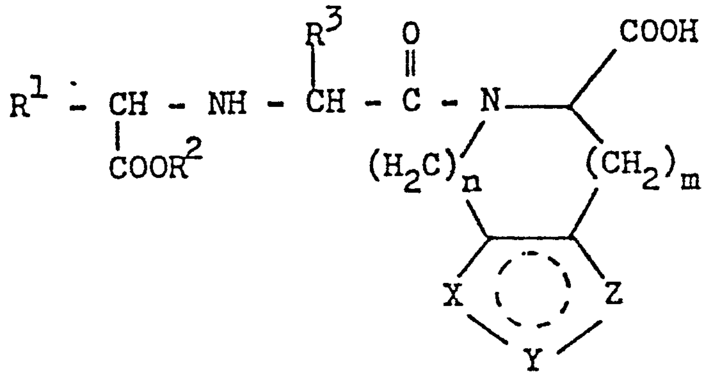

(I)

in der

$R^1$ Wasserstoff, eine Alkylgruppe mit 1 bis 6 Kohlenstoffatomen oder eine Phenylalkylgruppe mit 7 bis 12 Kohlenstoffatomen,

$R^2$ Wasserstoff, eine Alkylgruppe mit 1 bis 4 Kohlenstoffatomen oder eine Phenylalkylgruppe mit 7 bis 12 Kohlenstoffatomen,

$R^3$ Wasserstoff oder eine Alkylgruppe mit 1 bis 6 Kohlenstoffatomen,

n und m jeweils 0, 1 oder 2, wobei die Summe aus n und m 1 oder 2 ist,

X, Y und Z Sauerstoff, Schwefel, $NR^4$, $CR^5$, $CHR^5$, $-\overset{R^5}{\overset{|}{C}H}-\overset{R^5}{\overset{|}{C}H}-$ oder $-\overset{R^5}{\overset{|}{C}}=\overset{R^5}{\overset{|}{C}}-$ bedeutet, mit der Maßgabe, daß nur einer der Reste X, Y und Z Sauerstoff, Schwefel, $-\overset{R^5}{\overset{|}{C}H}-\overset{R^5}{\overset{|}{C}H}-$ oder $-\overset{R^5}{\overset{|}{C}}=\overset{R^5}{\overset{|}{C}}-$ und ein oder zwei der Reste X, Y und Z $NR^4$ bedeuten können,

$R^4$ Wasserstoff oder eine Alkylgruppe mit 1 - 4 Kohlenstoffatomen und

$R^5$ Wasserstoff oder zusammen mit einem vicinal stehenden Rest $R^5$ einen Phenylring bedeutet, oder, für m und n = 1, deren Dihydroform mit der Doppelbindung in Konjugation zur C-terminalen Carboxylgruppe und deren Salzen.

2. Verbindungen nach Anspruch 1, dadurch gekennzeichnet, daß alle Asymmetriezentren in der L-Form vorliegen.

3. Aminosäure-Derivate der allgemeinen Formel

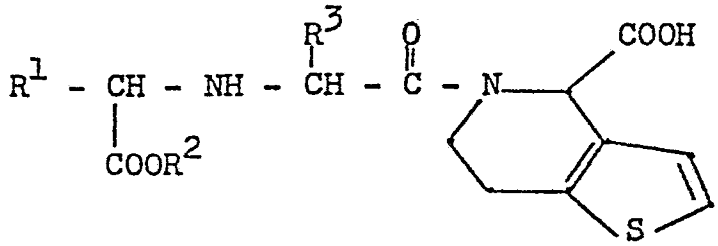

(Ia)

in der die Reste $R^1$, $R^2$ und $R^3$ die in Anspruch 1 angegebene Bedeutung besitzen sowie deren Salze.

4. Verbindungen nach Anspruch 3, dadurch gekennzeichnet, daß alle Asymmetriezentren in der L-Form vorliegen.

5. Aminosäure-Derivate der allgemeinen Formel

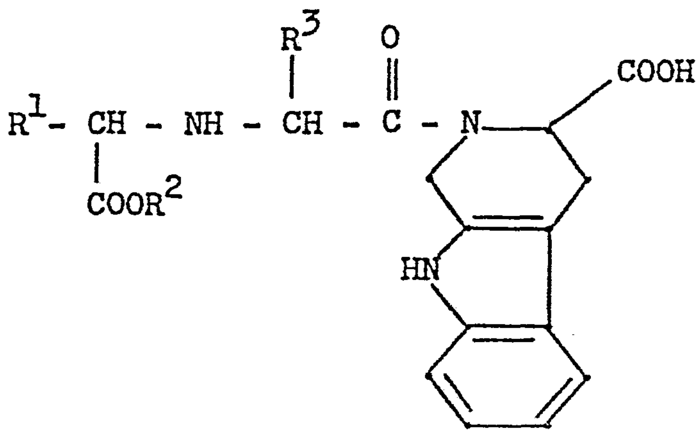

(Ib)

in der $R^1$, $R^2$ und $R^3$ die in Anspruch 1 angegebene Bedeutung besitzen sowie deren Salze.

6. Verbindungen nach Anspruch 5, dadurch gekennzeichnet, daß alle Asymmetriezentren in der L-Form vorliegen.

7. N-[N-(L-1-Ethoxycarbonyl-3-phenylpropyl)-L-alanyl]-4,5,6,7-tetrahydro-thieno[3,2-c]pyridin-L-4-carbonsäure.

8. N-[N-(L-1-Carboxy-3-phenylpropyl)-L-alanyl]-4,5,6,7-tetrahydro-thieno[3,2-c]pyridin-L-4-carbonsäure.

9. N-[N-(L-1-Ethoxycarbonyl-3-phenylpropyl)-L-alanyl]-4,5-dihydro-thieno[3,2-c]pyridin-6-carbonsäure.

10. Verfahren zur Herstellung neuer Aminosäure-Derivate der allgemeinen Formel I gemäß Anspruch 1 und deren Salze, dadurch gekennzeichnet, daß man

a) eine Verbindung der allgemeinen Formel

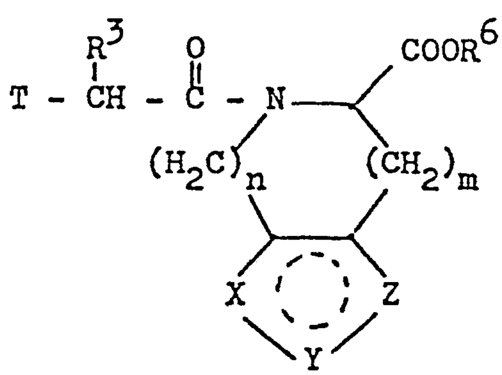

(II)

worin die Reste $R^3$, n, m, X, Y und Z die in Anspruch 1 angegebene Bedeutung haben,
T eine nucleofuge Gruppe und
U eine Aminogruppe oder
T eine Aminogruppe und U eine nucleofuge Gruppe und
$R^6$ Wasserstoff, eine Alkylgruppe mit 1 bis 4 Kohlenstoffatomen, eine Benzylgruppe oder eine Trimethylsilylgruppe bedeuten, mit einer Verbindung der allgemeinen Formel

$$R^1 - \underset{\displaystyle COOR^2}{\underset{|}{CH}} - U \qquad \text{(III)}$$

in der $R^1$, $R^2$ und U die oben angegebene Bedeutung besitzen, umsetzt, oder daß man

b) ein α-Oxocarbonsäure-Derivat der allgemeinen Formel

$$R^1 - \underset{\displaystyle COOR^2}{\underset{|}{C}} = O \qquad \text{(IV)}$$

in der $R^1$ und $R^2$ die oben angegebene Bedeutung besitzen, mit einer Verbindung der allgemeinen Formel III ($U = NH_2$) zum entsprechenden Imin umsetzt und dieses reduziert, oder daß man

c) eine Verbindung der allgemeinen Formel

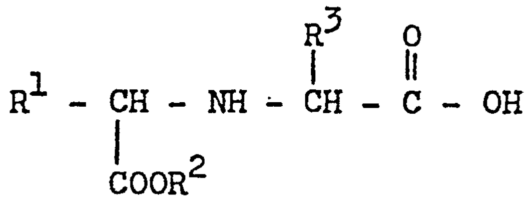

(V)

in der die Reste $R^1$, $R^2$ und $R^3$ die oben angegebene Bedeutung haben, mit einer Aminosäure der allgemeinen Formel

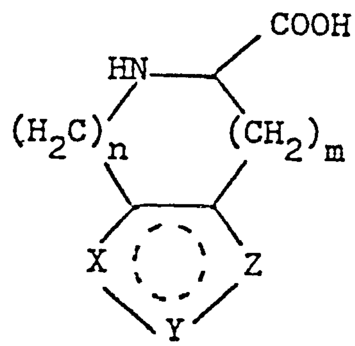

(VI)

in der X, Y, Z, n und m die oben angegebene Bedeutung haben, kondensiert,
und daß man ein so erhaltenes Endprodukt der allgemeinen Formel I nach üblichen Verfahren in ein physiologisch unbedenkliches Salz umwandelt.

11. Pharmazeutische Zubereitungen, enthaltend als Wirkstoff eine oder mehrere Verbindungen der Formel I oder deren Salze in Kombination mit üblichen Hilfs- und/oder Trägerstoffen.

12. Pharmazeutische Zubereitungen, enthaltend als Wirkstoff eine oder mehrere Verbindungen der allgemeinen Formel I in Gemisch mit anderen bekannten Saluretika beziehungsweise Diuretika und/oder Antihypertonika.

13. Verfahren zur Herstellung von pharmazeutischen Präparaten gemäß Anspruch 11, dadurch gekennzeichnet, daß man Verbindungen der allgemeinen Formel I mit üblichen galenischen Hilfs- und/oder Trägerstoffen zu üblichen pharmazeutischen Anwendungsformen verarbeitet.

14. Verwendung der Verbindungen nach Anspruch 1 als Hypotensiva.